# EUROPÄISCHE PATENTSCHRIFT

(11) **EP 0 998 474 B1**
(45) Veröffentlichungstag und Bekanntmachung des Hinweises auf die Patenterteilung: **07.02.2007**
(21) Anmeldenummer: 98938671.9
(22) Anmeldetag: 29.06.1998
(51) Int. Cl.: C07D 405/12, A61K 31/495

(54) **PIPERAZIN-DERIVATE**
PIPERAZINE DERIVATIVES
DERIVES DE PIPERAZINE

(30) Priorität: 18.07.1997 DE 19730989
(43) Veröffentlichungstag der Anmeldung: 10.05.2000
(73) Patentinhaber: Merck Patent GmbH, 64293 Darmstadt (DE)
(72) Erfinder: BÖTTCHER, Henning, D-64287 Darmstadt (DE); BARTOSZYK, Gerd, D-64331 Weiterstadt (DE); GREINER, Hartmut, D-64331 Weiterstadt (DE); SEYFRIED, Christoph, D-643432 Seeheim (DE)
(86) Internationale Anmeldenummer: PCT/EP1998/003956
(87) Internationale Veröffentlichungsnummer: WO 1999/003855

(56) Entgegenhaltungen:
- WO-A-98/04542
- FR-A- 2 189 027

## Beschreibung

Die Erfindung betrifft Piperazin-Derivate der Formel I 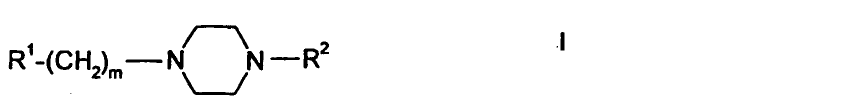 worin
- R¹: einen in 5-Position ein fach durch Hal, CN, COOH oder COOCH₃ substituierten Indol-3-yl-rest,
- R²: unsubstituiertes oder ein- oder zweifach durch A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH und/oder COOA substituiertes 2-Oxo-2H-1-benzopyran-6-yl oder 2-Oxo-2H-1-benzopyran-4-yl,
- Hal: F, Cl, Br oder I.
- A: geradkettiges oder verzweigtes Alkyl mit 1-10 C-Atomen, das durch 1 bis 5 F- und/oder Cl-Atome substituiert sein kann oder Cycloalkyl mit 3-10 C-Atomen,
- m: 2, 3 oder 4
bedeuten, sowie deren physiologisch unbedenklichen Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Säureadditionssalze wertvolle pharmakologische Eigenschaften besitzen. Die Verbindungen der Formel I beeinflussen die serotoninerge Transmission. Da die Verbindungen auch die SerotoninWiederaufnahme hemmen, eignen sie sich insbesondere als Antidepressiva und Anxiolytika. Die Verbindungen zeigen serotonin-agonistische und -antagonistische Eigenschaften. Sie hemmen die Bindung von tritiierten Serotoninliganden an hippocampale Rezeptoren (Cossery et al., European J. Pharmacol. 140 (1987), 143-155) und hemmen die synaptosomale Serotoninwiederaufnahme (Sherman et al., Life Sci. 23 (1978), 1863-1870). Außerdem treten Veränderungen der DOPA-Akkumulation im Striatum und der 5-HT-Akkumulation in verschiedenen Gehimregionen auf (Seyfried et al., European J. Pharmacol. 160 (1989), 31-41). Die 5-HT_{1A}-antagonistische Wirkung wird in vitro z.B. durch Hemmung der durch 8-OH-DPAT-verursachten Aufhebung der elektrisch induzierten Kontraktion des Meerschweinchenileums nachgewiesen (Fozard und Kilbinger, Br.J.Pharmacol. 86 (1985) 601 P). Ex-vivo dient zum Nachweis der 5-HT_{1A}-antagonistischen Wirkung die Hemmung der durch 8-OH-DPAT verminderten 5-HTP-Akkumulation (Seyfried et al., European J. Pharmacol. 160 (1989), 31-41) und die Antagonisierung der durch 8-OH-DPAT-induzierten Effekte im Ultraschallvokalisationstest (DeVry, Psychpharmacol. 121 (1995), 1-26). Zum ex-vivo Nachweis der Serotoninwiederaufnahmehemmung wird die synaptosomale Aufnahmehemmung (Wong et al., Neuropsychopharmacol. 8 (1993), 23-33) und der p-Chloramphetaminantagonismus (Fuller et al., J. Pharmacol. Exp. Ther. 212 (1980), 115-119) herangezogen. Weiterhin treten analgetische und blutdrucksenkende Wirkungen auf.

Die Verbindungen sind daher geeignet für die Behandlung von Schizophrenie, kognitiven Defiziten, Angst, Depressionen, Übelkeit, tardiver Dyskinesien, Magen-Darm-Trakt-Störungen, Lernstörungen, altersabhängigen Erinnerungsstörungen, Psychosen und zur positiven Beeinflussung von Zwangsverhalten (OCD) und Essstörungen (z.B. Bulimie). Sie zeigen Wirkungen auf das Zentralnervensystem, vor allem zusätzliche 5-HT_{1A}-agonistische und 5-HT-Reuptake hemmende Wirkungen. Ebenso eignen sie sich zur Prophylaxe und zur Bekämpfung der Folgen cerebraler Infarktgeschehen (apoplexia cerebri) wie Schlaganfall und cerebraler lschämien sowie zur Behandlung extrapyramidal-motorischer Nebenwirkungen von Neuroleptika sowie des Morbus Parkinson.

Die Verbindungen der Formel I eignen sich daher sowohl in der Veterinärals auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Sind sie jedoch auch geeignet als Arzneimittelwirkstoffe für Anxiolytika, Antidepressiva, Antipsychotika, Neuroleptika, Antihypertonika und/oder zur positiven Beeinflussung von Zwangsverhalten, Schlafstörungen, tardiver Dyskinesien, Lernstörungen, altersabhängiger Erinnerungsstörungen, Essstörungen wie Bulimie und/oder Sexualfunktionsstörungen.

Gegenstand der Erfindung sind Piperazinderivate der Formel I sowie ihre physiologisch unbedenklichen Säureadditionssalze.

Gegenstand der Erfindung sind insbesondere Verbindungen der Formel I, ausgewählt aus der Gruppe
a) 3-{4-[4-(2-Oxo-2H-1-benzopyran-6-yl)-1-piperazinyl]-butyl}-indol-5-carbonitril;
b) 3-{4-[4-(2-Oxo-2H-1-benzopyran-6-yl)-1-piperazinyl]-butyl}-5-fluor-indol;
c) 3-(4-[4-(2-Oxo-2H-1-benzopyran-4-yl)-1-piperazinyl]-butyl)-indol-5-carbonitril;
d) 3-{4-[4-(7-Hydroxy-2-oxo-2H-1-benzopyran-6-yl)-1-piperazinyl]-butyl}-indol-5-carbonitril;
sowie deren physiologisch unbedenklichen Salze.

Für alle Reste, die mehrfach auftreten, wie z.B. A, gilt, dass deren Bedeutungen unabhängig voneinander sind.

Der Rest A bedeutet Alkyl und hat 1 bis 10 , vorzugsweise 1, 2, 3, 4, 5 oder 6, insbesondere 1 oder 2 C-Atome. Alkyl bedeutet daher insbesondere z.B. Methyl, weiterhin Ethyl, n-Propyl, Isopropyl, n-Butyl, sek.-Butyl oder tert.-Butyl, femer auch Pentyl, 1-, 2- oder 3-Methylbutyl, 1,1-, 1,2- oder 2,2-Dirnethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2-oder 1,2,2-Trimethylpropyl, femer auch Fluormethyl, Difluormethyl, Trifluormethyl, 1,1,1-Trichlorethyl oder Pentafluorethyl.

Cycloalkyl bedeutet insbesondere z.B. Cyclopropyl, Cyclobutyl, Cyclopentyl, Cyclohexyl, Cycloheptyl öder 1-Adamantyl.

OA ist vorzugsweise Methoxy, ferner auch Ethoxy, n-Propoxy, lsopropoxy, n-Butoxy, lsobutoxy, sek.-Butoxy oder tert.-Butoxy. NHA ist vorzugsweise Methylamino, ferner Ethylamino, Isopropylamino, n-Butylamino, Isobutylamino, sek.-Butylamino oder tert.-Butylamino. NA₂ bedeutet vorzugsweise Dimethylamino, ferner N-Ethyl-N-methylamino, Diethylamino, Di-n-propylamino, Diisopropylamino oder Di-n-butylamino. Daraus resultierend bedeutet CO-NHA vorzugsweise N-Methylcarbamoyl oder N-Ethyl-carbamoyl; CO-NA₂ vorzugsweise N,N-Dimethylcarbamoyl oder N, N-Diethylcarbamoyl.

Hal bedeutet Fluor, Chlor, Brom oder lod, insbesondere Fluor oder Chlor. k bedeutet 0 oder 1, vorzugsweise 0. m bedeutet 1, 2, 3 oder 4, insbesondere 3 oder 4.

Der Rest R¹ bedeutet vorzugsweise einfach, durch Hal, CN, COOH oder COOCH₃ substituiertes 3-Indolyl. Vorzugsweise ist der Indolrest in 5-Stellung substituiert.

Der Rest R² bedeutet vorzugsweise unsubtituiertes oder einfach durch A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH und/oder COOA substituiertes 2-Oxo-2H-1-benzopyran-6-yl oder 2-Oxo-2H-1-benzopyran-4-yl.
Vorzugsweise kommen als Substituenten A, AO, OH, Hal, CN, NH₂, NHA, NA₂ oder auch CH₂OH in Frage.

R₂ bedeutet daher vorzugsweise 2-Oxo-2H-1-benzopyran-6-yl oder 2-Oxo-2H-1-benzopyran-4-yl, 7-Hydroxy-2-oxo-2H-1-benzopyran-6-yl, 7-Hydroxy-2-oxo-2H-1-benzopyran-4-yl, 7-Fluor-2-oxo-2H-1-benzopyran-6-yl, 7-Fluor-2-oxo-2H-1-benzopyran-4-yl, 5- Fluor-2-oxo-2H-1-benzopyran-6-yl, 6-Fluor-2-oxo-2H-1-benzopyran-4-yl, 5-Methyl-2-oxo-2H-1-benzopyran-4-yl, 7-Methyl-2-oxo-2H-1-benzopyran-6-yl, 7-Dimethylamino-2-oxo-2H-1-benzopyran-6-yl, 7-Hydroxymethyl-2-oxo-2H-1-benzopyran-6-yl oder auch 7-Chlor-2-oxo-2H-1-benzopyran-6-yl.

Für die gesamte Erfindung gilt, daß sämtliche Reste, die in einem Molekül mehrfach auftreten können, gleich oder verschieden sein können, d.h. unabhängig voneinander sind.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ib bis Ij ausgedrückt werden, die der Formel 1 entsprechen und worin die nicht näher bezeichneten Reste die bei der Formel 1 angegebene Bedeutung haben, worin jedoch
- in Ib: R¹ in 5-Position substituiertes 3-Indolyl ist;
- in Ic: k 0 und
m 4 bedeuten;
- in Id: k 1 und
m 3 bedeuten;
- in le: R¹ eine in Ib angegebene Bedeutung hat und der Substituent Hal, Methoxycarbonyl, CN oder Carboxy ist;
- in If: R² 2-Oxo-2H-1-benzopyran-6-yl ist;
- in lg: R² 2-Oxo-2H-1-benzopyran-4-yl ist;
- in Ih: R² ine in lf angegebene Bedeutung hat, wobei in Position 7 ein weiterer Substituent vorliegt;
- in li: R² eine in lg angegebene Bedeutung hat, wobei in Position 7 ein weiterer Substituent vorliegt;
- in Ij: R² eine in Ih oder Ii angegebene Bedeutung hat, und der Substituent Hal oder OH ist.

Gegenstand der Erfindung ist femer ein Verfahren zur Herstellung von Piperazin-Derivaten der Formel I sowie von deren Salzen, dadurch gekennzeichnet, dass man eine Verbindung der Formel II 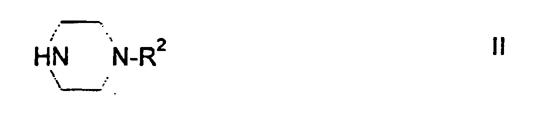 worin
- R²: die angegebene Bedeutung besitzt, mit einer Verbindung der Formel III

R¹-(CH₂)ₘ-(CO)ₖ-L III

worin
- L: Cl, Br, I, OH, OCOA, OCOPh, OSO₂A, OSO₂Ar, wobei Ar für Phenyl oder Tolyl und A für Alkyl stehen, oder eine andere reaktionsfähig veresterte OH-Gruppe oder leicht nucleophil substituierbare Abgangsgruppe bedeutet und
- R¹, m und k: die angegebenen Bedeutungen haben,
umsetzt,
oder dass man in einer reduktiven Aminierung eine Verbindung der Formel IV

R¹-(CH₂)ₘ₋₁-CHO IV

worin
R¹ und m die bereits angegebenen Bedeutungen haben, mit einer Verbindung der Formel II umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man gegebenenfalls einen Rest R¹ und/oder R² in einen anderen Rest R¹ und/oder R² umwandelt, indem man z.B. eine OA-Gruppe unter Bildung einer OH-Gruppe spaltet und/oder eine CN-, COOH- oder COOA-Gruppe derivatisiert und/oder daß man z.B. ein primäres oder sekundäres N-Atom alkyliert und/oder daß man eine erhaltene Base oder Säure der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York; DE-OS 41 01 686) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formeln III, V und VI sind die Reste L, X¹ und X² vorzugsweise Cl oder Br; sie können jedoch auch I, OH oder auch bevorzugt eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalinsulfonyloxy).

Die Ausgangsstoffe der Formeln II und III sind in der Regel bekannt; die nicht bekannten Verbindungen der Formeln II und III können leicht analog zu den bekannten Verbindungen hergestellt werden.

Die Piperazin-Derivate der Formel II sind größtenteils bekannt. Sofern sie nicht käuflich zu erwerben oder bekannt sind, können sie nach an sich bekannten Methoden hergestellt werden. Sie können beispielsweise durch Umsetzung von Bis-(2-chlorethyl)-amin oder Bis-(2-chlorethyl)-ammoniumchlorid mit Amino-substituierten Benzopyranverbindungen hergestellt werden.

Die Indol-Derivate der Formel III sind größtenteils bekannt und teilweise auch käuflich zu erwerben. Ferner kann man die Verbindungen durch elektrophile oder in bestimmten Fällen auch nukleophile aromatische Substitutionen aus bekannten Verbindungen herstellen. Als Ausgangssubstanz dient vorzugsweise eine entsprechende Indol-3-alkansäure (herstellbar analog einer Japp-Klingemann-Typ Fischer Indolsynthese, vgl. dazu Böttcher et al., J. Med. Chem. 1992, 35, 4020-4026 oder lyer et al., J. Chem. Soc. Perkin Trans. II 1973, 872-878). Primäre Alkohole der Formel R¹-(CH₂)ₘ-OH sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel R²-(CH₂)ₘ-Hal. Die entsprechenden Sulfonyloxyverbindungen sind erhältlich aus den Alkoholen durch Umsetzung mit den entsprechenden Sulfonsäurechloriden.

Die lodverbindungen der Formel R¹-(CH₂)ₘ-I sind z.B. durch Einwirkung von Kaliumiodid auf die zughörigen p-Toluolsulfonsäureester erhältlich. Die Amine der Formel R¹-(CH₂)ₘ-NH₂ sind z.B. aus den Halogeniden mit Phthalimidkalium oder durch Reduktion der entsprechenden Nitrile herstellbar.

Die Umsetzung der Verbindungen II und III verläuft nach Methoden, wie sie für die Alkylierung bzw. Acylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses Piperazin-Derivates der Formel II kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Gegebenenfalls ist es notwendig, vor der Durchführung dieser Reaktion weitere enthaltene Aminogruppen durch Einführung von geeigneten Schutzgruppen vor einer Alkylierung oder Acylierung zu schützen. Der Ausdruck Aminoschutzgruppe ist allgemein bekannt und bezieht sich auf Gruppen, die geeignet sind, eine Aminogruppe vor chemischen Umsetzungen zu schützen, die aber leicht entfembar sind, nachdem die gewünschte chemische Reaktion an einer anderen Stelle des Moleküls durchgeführt worden ist. Da dem Fachmann solche Schutzgruppen sowie die Einführung und Abspaltung dieser aus zahlreichen Literaturstellen und Lehrbüchern wohl bekannt sind, muss an dieser Stelle darauf nicht näher eingegangen werden.

Verbindungen der Formel I lassen sich weiterhin durch reduktive Aminierung von Verbindungen der Formel IV mit Verbindungen der Formel II erhalten. Die Ausgangsstoffe der Formeln IV und II sind teilweise bekannt. Sofern sie nicht bekannt sind, können sie nach an sich bekannten Methoden hergestellt werden. Die reduzierende Aminierung kann in Gegenwart von Reduktionsmitteln wie zum Beispiel NaBH₃CN und NaBH(OAc)₃ durchgeführt werden.

Es ist ferner möglich, eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels. Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Gruppe I, die in der Ausgangsverbindung enthalten sind, reduziert werden. Vorzugsweise bedient man sich hierzu des nascierenden Wasserstoffs oder komplexer Metallhydride, femer der Reduktion nach Wolff-Kishner sowie der Reduktionen mit Wasserstoffgas unter Übergangsmetallkatalyse.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall gelöst in einem Alkohol wie Ethanol, Isopropanol, Butanol, Amyl- oder Isoamylalkohol oder Phenol. Man kann femer eine Aluminium-Nickel-Legierung in alkalisch-wäßriger Lösung, gegebenenfalls unter Zusatz von Ethanol, verwenden. Auch Natrium- oder Aluminiumamalgam in wäßrig-alkoholischer oder wäßriger Lösung sind zur Erzeugung des nascierenden Wasserstoffs geeignet. Die Umsetzung kann auch in heterogener Phase durchgeführt werden, wobei man zweckmäßig eine wäßrige und eine Benzol- oder Toluol-Phase verwendet.

Als Reduktionsmittel können femer besonders vorteilhaft komplexe Metallhydride, wie LiAlH₄, NaBH₄, Düsobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für die Reduktion mit NaBH₄ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150°, insbesondere zwischen etwa 0 und etwa 100°.

Darüber hinaus ist es möglich, bestimmte Reduktionen durch Verwendung von H₂-Gas unter katalytischer Wirkung von Übergangsmetallen, wie z.B. Raney-Ni oder Pd durchzuführen. Man kann auf diese Weise z.B. Cl, Br, I, SH oder in bestimmten Fällen auch OH-Gruppen durch Wasserstoff ersetzen. Ebenso können Nitrogruppen durch katalytische Hydrierung mit Pd/H₂ in Methanol in NH₂-Gruppen umgewandelt werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Weiterhin kann man eine Verbindung der Formel I nach an sich bekannten Methoden in eine andere Verbindung der Formel I umwandeln.

Verbindungen der Formel I, worin R¹ einen durch CONH₂, CONHA oder CONA₂ substituierten Rest bedeutet, können durch Derivatisierung entsprechender substituierter Verbindungen der Formel I durch partielle Hydrolyse erhalten werden. Ferner ist es möglich, Cyan-substituierte Verbindungen der Formel I zunächst zu Säuren zu hydrolysieren und die Säuren mit primären oder sekundären Aminen zu amidieren. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-Dimethylaminopropyl)-N-ethyl-carbodiimid, ferner Propanphosphonsäureanhydrid (vgl. Angew. Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40°, vorzugsweise zwischen 0 und 30°. Anstelle der Säure bzw. des Amids können auch reaktionsfähige Derivate dieser Stoffe in die Reaktion eingesetzt werden, z.B. solche, in denen reaktive Gruppen intermediär durch Schutzgruppen blockiert sind.

Die Säuren können auch in Form ihrer aktivierten Ester verwendet werden, die zweckmäßig in situ gebildet werden, z.B. durch Zusatz von 1-Hydroxybenztriazol oder N-Hydroxysuccinimid.

So können auch beispielweise cyan-substituierte Indolreste zu Carboxyindol- oder Carboxamido-indolreste hydrolysiert werden.

Besonders günstig ist es aber auch in umgekehrter Weise, durch Wasserabspaltung, ausgehend von den Amiden, z.B. mittels Trichloracetylchlorid/Et₃N (Synthesis (2), 184 (1985) oder mit POCl₃ (J. Org. Chem. 26, 1003 (1961)), die Nitrile herzustellen.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, femer organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bemsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure; p-Toluolsulfonsäure; Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässerige Lösungen, femer Suspensionen, Emulsionen oder Implantate, für die topische Anwendung Salben, Cremes oder Puder. Die neuen Verbindungen können auch lyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich zur Behandlung von Erkrankungen des Zentralnervensystems wie Spannungszuständen, Depressionen, Angstzuständen, Schizophrenie, Magen-Darm-Trakt-Störungen, Übelkeit, tardiven Dyskinesien, Parkinsonismus und/oder Psychosen und von Nebenwirkungen bei der Behandlung der Hypertonie (z.B. mit α-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide.

Insbesondere bevorzugt können sie auch als Therapeutika zur Bekämpfung der Folgen cerebraler Infarktgeschehen (apoplexia cerebri), wie Schlaganfall und cerebraler Ischämien und zur Behandlung von Hirn- und Rückenmarkstraumata eingesetzt werden.

Insbesondere sind sie jedoch geeignet als Arnzeimitelwirkstoffe für Anxiolxytika, Antidepresiva, Antipsychotika und/oder zur positiven Beeinflussung von Zwangsverhalten (OCD), Schlafstörungen, tardiver Dyskinesien, Lernstörungen, altersabhängiger Erinnerungsstörungen, Essstörungen wie Bulimie und/oder Sexualfunktionsstörungen.

Dabei werden die erfindungsgemäßen Substanzen in der Regel in Analogie zu bekannten, im Handel befindlichen Präparaten (z.B. Bromocriptin, Dihydroergocornin) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 und 10 mg/kg Körpergewicht. Die niedrigen Dosierungen liegen zwischen etwa 0,2 und 500 mg, insbesondere zwischen 0,2 und 50 mg pro Dosierungseinheit. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg pro Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachstehenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, filtriert, dampft ein und reinigt durch Chromatographie an Kieselgel und/oder durch Kristallisation. R_{f}-Werte wurden dünnschichtchromatographisch an Kieselgel erhalten. M⁺+1-Werte wurden durch FAB-MS (Fast-Atom-Bombardment-Massen-Spektroskopie) ermittelt.

### Beispiel 1

Man löst 0.79 g (0.003 mol) 4-(2-Oxo-2H-1-benzopyran-4-yl)-piperazin [erhältlich z.B. durch Umsetzung von N,N-Bis-(2-chlorethyl)-amin mit 4-Amino-2-oxo-2H-1-benzopyran] und 0.80 g (0.003 mol) 3-(4-Chlorbutyl)-5-cyan-indol [welche durch Reduktion von 3-(4-Chlorbutanoyl)-indol-5-carbonitril hergestellt werden kann] in 100 ml Acetonitril, gibt 0.50 ml (0.004 mol) Triethylamin sowie 1.20 ml (0.007 mol) Ethyldiisopropylamin zu und rührt über Nacht auf dem Dampfbad. Nach üblicher Aufarbeitung erhält man 3-{4-[4-(2-Oxo-2H-1-benzopyran-4-yl)-1-piperazinyl]-butyl}-indol-5-carbonitril, Dihydrochlorid, F. 284-285°.

Analog werden hergestellt:
3-{4-[4-(2-Oxo-2H-1-benzopyran-4-yl)-1-piperazinyl]-butyl}-5-fluor-indol,
3-{4-[4-(2-Oxo-2H-1-benzopyran-4-yl)-1-piperazinyl]-butyl}-5-chlor-indol.
3-{4-[4-(2-Oxo-2H-1-benzopyran-4-yl)-1-piperazinyl]-butyl}-indol-5-carbonsäuremethylester.

### Beispiel 2

Ein Gemisch aus 8.3 g (0.031 mol) 4-(2-Oxo-2H-1-benzopyran-6-yl)-piperazin, Hydrochlorid (Herstellung wie in Beispiel 1 erwähnt), 7.70 g (0.033 mol) 3-(4-Chlorbutyl)-5-cyan-indol (Herstellung siehe Beispiel 1), 6.7 g (0.066 mol) Triethylamin, 11.3 ml (0.066 mol) Ethyl-diisopropylamin und 55 ml 1-Methyl-2-pyrrolidon wird über Nacht bei einer Badtemperatur von 120-130° gerührt. Die Suspension wird dann in 4 l Eiswasser eingerührt und man erhält nach längerem Rühren kristallines 3-{4-[4-(2-Oxo-2H-1-benzopyran-6-yl)-1-piperazinyl]-butyl}-indol-5-carbonitril, F. 135-137°, als Dihydrochlorid, F. 282-284°.

Analog werden hergestellt:
3-{4-[4-(2-Oxo-2H-1-benzopyran-6-yl)-1-piperazinyl]-butyl}-indol-5-carbonitril, Monohydrochlorid, F. 287-290°,
3-(4-[4-(2-Oxo-2H-1-benzopyran-6-yl)-1-piperazinyl]-butyl)-indol-5-carbonsäuremethylester.

### Beispiel 3

Ein Gemisch aus 5.10 g (0.017 mol) Methansulfonsäure-4-(5-fluor-indol-3-yl)-butylester [erhältlich durch Umsetzung von 4-(5-Fluor-indol-3-yl)-butanol (erhältlich durch Reduktion mit Lithiumaluminiumhydrid von 4-(5-Fluor-indol-3-yl)-butansäure, die analog einer Japp-Klingemann-Reaktion dargestellt werden kann, in THF) mit Methansulfonsäurechlorid], 4.0 g (0.015 mol) 4-(2-Oxo-2H-1-benzopyran-6-yl)-piperazin, Hydrochlorid (erhältlich wie in Beispiel 1 beschrieben), 200 ml Acetonitril und 10.0 ml Triethylamin wird 30 Stunden lang auf dem Dampfbad unter Rühren umgesetzt. Nach üblicher Aufarbeitung erhält man 3-{4-[4-(2-Oxo-2H-1-benzopyran-6-yl)-1-piperazinyl]-butyl}-5-fluor-indol, Hydrochlorid, F. 293-295°.

Analog werden hergestellt:
3-{4-[4-(2-Oxo-2H-1-benzopyran-6-yl)-1-piperazinyl]-butyl}-5-chior-indol,
3-{4-[4-(7-Hydroxy-2-oxo-2H-1-benzopyran-6-yl)-1-piperazinyl]-butyl}-5-fluor-indol,
3-{4-[4-(7-Hydroxy-2-oxo-2H-1-benzopyran-6-yl)-1-piperazinyl]-butyl}-5-chlor-indol.

### Beispiel 4

Ein Gemisch aus 0.0098 Mol Methansulfonsäure-4-(5-methoxycarbonyl-indol-3-yl)-butylester (Herstellung wie in Beispiel 3 beschrieben) und 0.0097 Mol 4-(2-Oxo-2H-1-benzopyran-6-yl)-piperazin, wird in Acetonitril ca. 96 Stunden auf dem Dampfbad erhitzt. Das Reaktionsgemisch wird wie üblich aufgearbeitet und gereinigt. Man erhält so den 3-{4-[4-(2-Oxo-2H-1-benzopyran-6-yl)-1-piperazinyl]-butyl}-indol-5-carbonsäuremethylester.

Analog erhält man:
3-{4-[4-(7-Methyl-2-oxo-2H-1-benzopyran-6-yl)-1-piperazinyl]-butyl}-indol-5-carbonsäuremethylester
3-{4-[4-(7-Methoxy-2-oxo-2H-1-benzopyran-6-yl)-1-piperazinyl]-butyl}-indol-5-carbonsäuremethylester
3-{4-[4-(7-Fluor-2-oxo-2H-1-benzopyran-6-yl)-1-piperazinyl]-butyl}-indol-5-carbonsäuremethylester
3-{4-[4-(7-Chlor-2-oxo-2H-1-benzopyran-6-yl)-1-piperazinyl]-butyl}-indol-5-carbonsäuremethylester
3-{4-[4-(7-Cyan-2-oxo-2H-1-benzopyran-6-yl)-1-piperazinyl]-butyl}-indol-5-carbonsäuremethylester
3-{4-[4-(7-Methyi-2-oxo-2H-1-benzopyran-4-yl)-1-piperazinyl]-butyl}-indoi-5-carbonsäuremethylester
3-{4-[4-(7-Methoxy-2-oxo-2H-1-benzopyran-4-yl)-1-piperazinyl]-butyl}-indol-5-carbonsäuremethylester
3-{4-[4-(7-Fluor-2-oxo-2H-1-benzopyran-4-yl)-1-piperazinyl]-butyl}-indol-5-carbonsäuremethylester
3-{4-[4-(7-Chlor-2-oxo-2H-1-benzopyran-4-yl)-1-piperazinyl]-butyl}-indol-5-carbonsäuremethylester
3-{4-[4-(7-Cyan-2-oxo-2H-1-benzopyran-4-yl)-1-piperazinyl]-butyl}-indol-5-carbonsäuremethytester.

### Beispiel 5

Man kocht 1.8 g 3-{4-[4-(2-Oxo-2H-1-benzopyran-6-yl)-1-piperazinyl]-butyl}-indol-5-carbonsäuremethylester 30 Minuten mit 100 ml 2n ethanlischer KOH, arbeitet wie üblich auf und erhält 3-{4-[4-(2-Oxo-2H-1-benzopyran-6-yl)-1-piperazinyl]-butyl}-indol-5-carbonsäure.

Analog erhält man:
3-{4-[4-(2-Oxo-2H-1-benzopyran-4-yl)-1-piperazinyl]-butyl}-indol-5-carbonsäure
3-{4-[4-(7-Methyl-2-oxo-2H-1-benzopyran-6-yl)-1-piperazinyl]-butyl}-indol-5-carbonsäure
3-{4-[4-(7-Methoxy-2-oxo-2H-1-benzopyran-6-yl)-1-piperazinyl]-butyl}-indol-5-carbonsäure
3-{4-[4-(7-Fluor 2-oxo-2H-1-benzopyran-6-yl)-1-piperazinyl]-butyl}-indol-5-carbonsäure
3-{4-[4-(7-Chlor-2-oxo-2H-1-benzopyran-6-yl)-1-piperazinyl]-butyl}-indol-5-carbonsäure
3-{4-[4-(7-Cyan-2-oxo-2H-1-benzopyran-6-yl)-1-piperazinyl]-butyl}-indol-5-carbonsäure
3-{4-[4-(7-Methyl-2-oxo-2H-1-benzopyran-4-yl)-1-piperazinyl]-butyl}-indol-5-carbonsäure
3-{4-[4-(7-Methoxy-2-oxo-2H-1-benzopyran-4-yl)-1-piperazinyl]-butyl}-indol-5-carbonsäure
3-{4-[4-(7-Fluor-2-oxo-2H-1-benzopyran-4-yl)-1-piperazinyl]-butyl}-indol-5-carbonsäure
3-{4-(4-(7-Chlor-2-oxo-2H-1-benzopyran-4-yl)-1-piperazinyl)-butyl}-indol-5-carbonsäure
3-{4-[4-(7-Cyan-2-oxo-2H-1-benzopyran-4-yl)-1-piperazinyl]-butyl}-indol-5-carbonsäure.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat in 3 I zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9.38 g NaH₂PO₄ x 2 H₂O, 28.48 g NaH₂PO₄ x 12 H₂O und 0.1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Verbindungen der Formel I 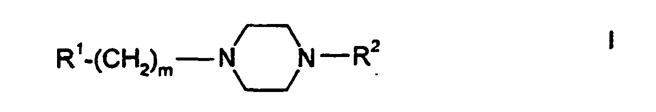 worin
R¹ einen in 5-Position ein fach durch Hal, CN, COOH, oder COOCH₃ substituierten Indol-3-yl-rest,
R² unsubstituiertes oder ein- oder zweifach durch A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH und/oder COOA substituiertes 2-Oxo-2H-1-benzopyran-6-yl oder 2-Oxo-2H-1-benzopyran-4-yl,
Hal F, Cl, Br oder I,
A geradkettiges oder verzweigtes Alkyl mit 1-10 C-Atomen, das durch 1 bis 5 F- und/oder Cl-Atome substituiert sein kann oder Cycloalkyl mit 3-10 C-Atomen,
m 2, 3 oder 4
bedeuten, sowie deren physiologisch unbedenklichen Salze.

2. Verbindungen nach Anspruch 1
a) 3-{4-[4-(2-Oxo-2H-1-benzopyran-6-yl)-1-piperazinyl]-butyl}-indol-5-carbonitril;
b) 3-{4-[4-(2-Oxo-2H-1-benzopyran-6-yl)-1-piperazinyl]-butyl}-5-fluor-indol;
c) 3-{4-[4-(2-Oxo-2H-1-benzopyran-4-yl)-1-piperazinyl]-butyl}-indol-5-carbonitril;
d) 3-{4-[4-(7-Hydroxy-2-oxo-2H-1-benzopyran-6-yl)-1-piperazinyl]-butyl}-indol-5-carbonitril;
sowie deren physiologisch unbedenklichen Salze.

3. Verfahren zur Herstellung von Piperazin-Derivaten der Formel I gemäß Anspruch 1 sowie von deren Salzen, **dadurch gekennzeichnet, daß** man eine Verbindung der Formel II 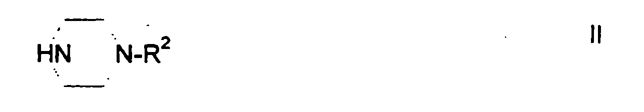 worin
R² die angegebene Bedeutung besitzt, mit einer Verbindung der Formel III
R¹-(CH₂)ₘ-(CO)ₖ-L III
worin
L Cl, Br, I, OH, OCOA, OCOPh, OSO₂A, OSO₂Ar, wobei Ar für Phenyl oder Tolyl und A für Alkyl stehen, oder eine andere reaktionsfähig veresterte OH-Gruppe oder leicht nucleophil substituierbare Abgangsgruppe bedeutet und
R¹, m und k die angegebenen Bedeutungen haben,
umsetzt,
oder dass man in einer reduktiven Aminierung eine Verbindung der Formel IV
R¹-(CH₂)ₘ₋₁-CHO IV
worin
R¹ und m die bereits angegebenen Bedeutungen haben, mit einer Verbindung der Formel II umsetzt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C- und/oder C-N-Bindung(en) enthält, mit einem reduzierenden Mittel behandelt,
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt,
und/oder daß man gegebenenfalls einen Rest R¹ und/cder R² in einen anderen Rest R¹ und/oder R² umwandelt, indem man z.B. eine OA-Gruppe unter Bildung einer OH-Gruppe spaltet und/oder eine CN-, COOH- oder COOA-Gruppe derivatisiert und/oder daß man z.B. ein primäres oder sekundäres N-Atom alkyliert und/oder daß man eine erhaltene Base oder Säure der Formel I durch Behandeln mit einer Säure oder Base in eines ihrer Salze umwandelt.

4. Verfahren zur Herstellung pharmazeutischer Zubereitungen, **dadurch gekennzeichnet, daß** man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

5. Pharmazeutische Zubereitung, **gekennzeichnet durch** einen Gehalt an mindestens einer Verbindung der allgemeinen Formel I und/oder einem ihrer physiologisch unbedenklichen Salze.

6. Verwendung von Verbindungen der Formel I nach Patentanspruch 1 oder von deren physiologisch unbedenklichen Salzen zur Herstellung eines Arzneimittels.

## Claims

1. Compounds of the formula I 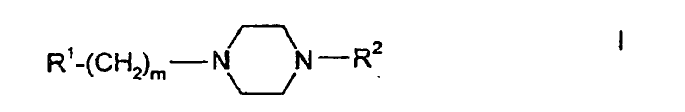 in which
R¹ denotes an indol-3-yl radical which is monosubstituted in the 5-position by Hal, CN, COOH or COOCH₃,
R² denotes 2-oxo-2H-1-benzopyran-6-yl or 2-oxo-2H-1-benzo-pyran-4-yl, each of which is unsubstituted or mono- or disubstituted by A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH and/or COOA,
Hal denotes F, Cl, Br or I,
A denotes straight-chain or branched alkyl having 1-10 C atoms, which may be substituted by 1 to 5 F and/or Cl atoms, or cyclo-alkyl having 3-10 C atoms,
m denotes 2, 3 or 4,
and physiologically acceptable salts thereof.

2. Compounds according to Claim 1
a) 3-{4-[4-(2-oxo-2H-1-benzopyran-6-yl)-1-piperazinyl]butyl}indole-5-carbonitrile;
b) 3-{4-[4-(2-oxo-2H-1-benzopyran-6-yl)-1-piperazinyl]butyl}-5-fluoroindole;
c) 3-{4-[4-(2-oxo-2H-1-benzopyran-4-yl)-1-piperazinyl]butyl}indole-5-carbonitrile;
d) 3-{4-[4-(7-hydroxy-2-oxo-2H-1-benzopyran-6-yl)-1-piperazinyl]-butyl}indole-5-carbonitrile;
and physiologically acceptable salts thereof.

3. Process for the preparation of piperazine derivatives of the formula I according to Claim 1 and of salts thereof, **characterised in that** a compound of the formula II 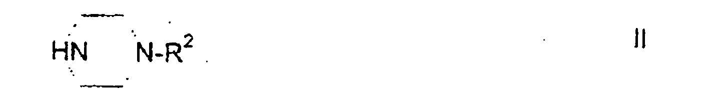 in which
R² has the meaning indicated, is reacted with a compound of the formula III
R¹-(CH₂)ₘ-(CO)ₖ-L III
in which
L denotes Cl, Br, I, OH, OCOA, OCOPh, OSO₂A, OSO₂Ar, where Ar stands for phenyl or tolyl and A stands for alkyl, or another reactively esterified OH group or readily nucleophilically substitutable leaving group and
R¹, m and k have the meanings indicated,
or **in that**, in a reductive amination, a compound of the formula IV
R¹-(CH₂)ₘ₋₁-CHO IV
in which
R¹ and m have the meanings already indicated, is reacted with a compound of the formula II,
or **in that** a compound which otherwise conforms to the formula I, but which contains one or more reducible group(s) instead of one or more hydrogen atoms and/or contains one or more additional C-C and/or C-N bond(s), is treated with a reducing agent,
or **in that** a compound which otherwise conforms to the formula I, but which contains one or more solvolysable group(s) instead of one or more hydrogen atoms, is treated with a solvolysing agent,
and/or **in that** a radical R¹ and/or R² is optionally converted into another radical R¹ and/or R² by, for example, cleaving an OA group with formation of an OH group and/or derivatising a CN, COOH or COOA group and/or **in that**, for example, a primary or secondary N atom is alkylated and/or **in that** a resultant base or acid of the formula I is converted into one of its salts by treatment with an acid or base.

4. Process for the preparation of pharmaceutical compositions, **characterised in that** a compound of the formula I and/or one of its physiologically acceptable salts is brought into a suitable dosage form together with at least one solid, liquid or semi-liquid excipient or adjuvant and optionally in combination with one or more further active ingredients.

5. Pharmaceutical composition, **characterised by** a content of at least one compound of the general formula I and/or one of its physiologically acceptable salts.

6. Use of compounds of the formula I according to Patent Claim 1 or of physiologically acceptable salts thereof for the preparation of a medicament.

## Revendications

1. Composés de la formule I 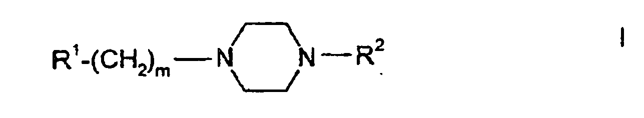 dans laquelle
R¹ représente un radical indol-3-yle qui est monosubstitué à la position 5 par Hal, CN, COOH ou COOCH₃,
R² représente 2-oxo-2H-1-benzopyrane-6-yle ou 2-oxo-2H-1-benzopyrane-4-yle, dont chacun est non substitué ou mono- ou disubstitué par A, AO, OH, Hal, CN, NO₂, NH₂, NHA, NA₂, COA, CONH₂, CONHA, CONA₂, CH₂OH, CH₂OA, CH₂NH₂, CH₂NHA, CH₂NA₂, COOH et/ou COOA,
Hal représente F, CI, Br ou I,
A représente alkyle en chaîne droite ou ramifié ayant 1-10 atomes de C, qui peut être substitué par 1 à 5 atomes de F et/ou de CI, ou cycloalkyle ayant 3-10 atomes de C,
m représente 2, 3 ou 4,
et leurs sels physiologiquement acceptables.

2. Composés selon la revendication 1
a) 3-{4-[4-(2-oxo-2H-1-benzopyrane-6-yl)-1-pipérazinyl]butyl}-indole-5-carbonitrile ;
b) 3-{4-[4-(2-oxo-2H-1-benzopyrane-6-yl)-1-pipérazinyl]butyl}-5-fluoroindole ;
c) 3-{4-[4-(2-oxo-2H-1-benzopyrane-4-yl)-1-pipérazinyl]-butyl}-indole-5-carbonitrile ;
d) 3-{4-[4-(7-hydroxy-2-oxo-2H-1-benzopyrane-6-yl)-1-pipérazinyl]-butyl}indole-5-carbonitrile ;
et leurs sels physiologiquement acceptables.

3. Procédé pour la préparation de dérivés de pipérazine de la formule I selon la revendication 1 et de leurs sels, **caractérisé en ce qu'**un composé de la formule II 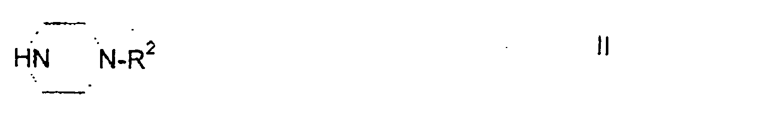 dans laquelle
R² a la signification indiquée, est amené à réagir avec un composé de la formule III
R¹-(CH₂)ₘ-(CO)ₖ-L III
dans laquelle
L représente CI, Br, I, OH, OCOA, OCOPh, OSO₂A, OSO₂Ar, où Ar représente phényle ou tolyle et A représente alkyle, ou un autre groupe OH estérifié de façon réactive ou un autre groupe partant substituable réellement nucléophiliquement et
R¹, m et k ont la signification indiquée,
ou **en ce que**, lors d'une amination réductive, un composé de la formule IV
R¹-(CH₂)ₘ₋₁-CHO IV
dans laquelle
R¹ et m ont les significations déjà indiquées, est amené à réagir avec un composé de la formule II,
ou **en ce qu'**un composé, qui est conforme de toute autre façon à la formule I, mais qui contient un ou plusieurs groupe(s) réductible(s) à la place d'un ou de plusieurs atome(s) d'hydrogène et/ou qui contient une ou plusieurs liaison(s) C-C et/ou C-N additionnelle(s), est traité avec un agent réducteur,
ou **en ce qu'**un composé, qui est conforme de toute autre façon à la formule I, mais qui contient un ou plusieurs groupe(s) solvolysable(s) à la place d'un ou de plusieurs atome(s) d'hydrogène, est traité avec un agent solvolysant,
et/ou **en ce qu'**un radical R¹ et/ou R² est en option converti selon un autre radical R¹ et/ou R², par exemple, en clivant un groupe OA avec formation d'un groupe OH et/ou en dérivant un groupe CN, COOH ou COOA et/ou **en ce que**, par exemple, un atome de N primaire ou secondaire est alkylaté et/ou **en ce qu'**une base ou un acide résultant de la formule I est converti selon l'un de ses sels par traitement avec un acide ou une base.

4. Procédé pour la préparation de compositions pharmaceutiques, **caractérisé en ce qu'**un composé de la formule I et/ou l'un de ses sels physiologiquement acceptables sont amenés selon une forme de dosage appropriée en association avec au moins un excipient ou adjuvant solide, liquide ou semi-liquide et en option, en combinaison avec un ou plusieurs autres ingrédients actifs.

5. Composition pharmaceutique, **caractérisée par** une teneur en au moins un composé de la formule générale I et/ou en l'un de ses sels physiologiquement acceptables.

6. Utilisation de composés de la formule I selon la revendication 1 ou de ses sels physiologiquement acceptables pour la préparation d'un médicament.
